# EUROPEAN PATENT SPECIFICATION

(11) **EP 2 145 950 B1**
(45) Date of publication and mention of the grant of the patent: **22.02.2017**
(21) Application number: 09008455.9
(22) Date of filing: 29.06.2009
(51) Int. Cl.: C12M 1/00

(54) **Culturing system**
Kultivierungssystem
Système de culture

(30) Priority: 08.07.2008 JP 2008178038
(43) Date of publication of application: 20.01.2010
(73) Proprietor: Olympus Corporation, Tokyo 192-8507 (JP)
(72) Inventor: Kimura, Hiroyuki, Tokyo 192-8507 (JP)
(74) Representative: Schicker, Silvia

(56) References cited:
- EP-A1- 1 548 484
- DE-A1- 10 128 811
- US-A- 5 181 382
- US-A1- 2006 023 299

## Description

### BACKGROUND OF THE INVENTION

### 1. FIELD OF THE INVENTION

The present invention relates to culturing systems.

### 2. DESCRIPTION OF RELATED ART

In a known simplified CO₂ incubator in the related art, a biological specimen is contained together with a culture solution to examine the specimen while maintaining the activity thereof over a long period of time see WO2004021066.

This CO₂ incubator has tubes provided so as to extend through a lid member to, for example, circulate the culture solution inside.

In the CO₂ incubator disclosed in the above publication, however, because the tubes are directly connected to, for example, circulate the culture solution, pulsations for circulating the culture solution are transmitted to the CO₂ incubator via the tubes. This causes a problem in that the specimen contained in the CO₂ incubator is vibrated, and accordingly, an observation image is vibrated.

EP 1 548 848 A1 discloses an incubator for observing a specimen in a dish by a microscope while culturing the specimen. Light is radiated from a capacitor, passed through a hole in a top plate, a hole in a upper side plate and a hole in a lower side plate, and let into an objective lens, and the specimen put in the dish is observed by the microscope, The change of the specimen with elapse of time can be continuously observed or recorded on a videotape while culturing the observed specimen in the dish.

### BRIEF SUMMARY OF THE INVENTION

An object of the present invention, which has been made in light of the above circumstances, is to provide a culturing system that can avoid the problem of a specimen being vibrated during supply or drainage of a liquid such as a culture solution, thus allowing acquisition of a stable image.

To achieve the above object, the present invention provides a culturing system as defined in claim 1.

An aspect of the present invention is a culturing system that includes a main container having an opening facing upward, accommodating a culture vessel containing a biological specimen, and having an inner space maintained at a predetermined incubation environment; and a nozzle fixed to the main container and inserted into the culture vessel through the opening of the culture vessel to supply or drain a liquid into or from the culture vessel. The main container includes a transparent portion through which the biological specimen in the main container can be externally observed.

According to the above aspect, it is possible to accommodate the culture vessel in the main container, to maintain the inner space of the main container at the predetermined incubation environment, and to observe changes over time in the biological specimen in the main container through the transparent portion while supplying or draining
the liquid into or from the culture vessel through the nozzle. In this case, the nozzle is not in direct contact with the culture vessel because the nozzle is fixed to the main container with the end thereof inserted into the culture vessel. This inhibits transmission of, for example, pulsations of a pump occurring in the nozzle during the supply or drainage of the liquid through the nozzle to the culture vessel, thus preventing vibration of the biological specimen in an observation image.

In the above aspect, the main container may include a base member having an opening facing upward and a lid member sealing off the opening of the base member, the base member may include a bottom portion on which the culture vessel is mounted and a peripheral wall extending upward from the bottom portion, and the nozzle may be fixed to the lid member.

In this case, because the nozzle is fixed to the lid member, which is separated from the base member with the culture vessel mounted on the bottom portion thereof, the transmission of vibrations of the nozzle to the culture vessel can be further inhibited, thus allowing more stable examination.

In the above configuration, a damper may be disposed between the base member and the lid member to inhibit transmission of vibrations therebetween.

In this case, the damper inhibits the transmission of vibrations between the base member and the lid member. Even if the lid member is vibrated by pulsations of the nozzle during the supply or drainage of the liquid, the damper can inhibit transmission of the vibrations to the base member and the culture vessel, thus allowing stable examination as a result of alleviated vibration of the biological specimen.

In the above aspect, a damper may be disposed between the main container and the nozzle to inhibit transmission of vibrations therebetween.

In this case, the damper inhibits the transmission of vibrations between the main container and the nozzle. Even if the nozzle experiences pulsations during the supply or drainage of the liquid, the damper can inhibit transmission of the vibrations to the main container, thus allowing stable examination as a result of alleviated vibration of the biological specimen.

In the above configuration, a support may be provided on the bottom portion of the base member so as to support the culture vessel, and a damper may be disposed between the support and the culture vessel to inhibit transmission of vibrations therebetween.

In this case, the damper inhibits the transmission of vibrations between the support and the culture vessel. Even if the support is vibrated during the supply or drainage of the liquid, the damper can inhibit transmission of the vibrations to the culture vessel, thus allowing stable examination as a result of alleviated vibration of the biological specimen.

In the above configuration, a support may be provided on the bottom portion of the base member so as to support the culture vessel in contact with the underside of the culture vessel.

Some types of culture vessels have a leg portion for raising the bottom portion on the periphery of the bottom portion. If the height of the leg portion varies depending on the type of culture vessel, the level of the biological specimen varies accordingly.

The above configuration, in which the support is disposed so as to support the culture vessel in contact with the underside thereof, prevents variations in the level of the biological specimen under examination for different types of culture vessels. This allows the level of the nozzle to be kept constant relative to that of the biological specimen without adjusting the level of the nozzle.

In the above aspect, the nozzle may be attachably/detachably attached to the main container.

In this case, it is possible to select and attach/detach a nozzle matching the type of biological specimen incubated and the incubation conditions thereof.

The present invention provides the advantage of preventing the problem of a specimen being vibrated during supply or drainage of a liquid such as a culture solution, thus allowing acquisition of a stable image.

### BRIEF DESCRIPTION OF THE SEVERAL VIEWS OF THE DRAWINGS

Fig. 1 is a longitudinal sectional view showing a culturing system according to an embodiment of the present invention.
Fig. 2 is a partial longitudinal sectional view showing an example of a nozzle attachment structure of the culturing system in Fig. 1.
Fig. 3 is a longitudinal sectional view showing a first modification of the culturing system in Fig. 1.
Fig. 4 is a longitudinal sectional view showing a second modification of the culturing system in Fig. 1.
Fig. 5 is a longitudinal sectional view showing a third modification of the culturing system in Fig. 1.
Fig. 6 is a longitudinal sectional view showing a fourth modification of the culturing system in Fig. 1.

### DETAILED DESCRIPTION OF THE INVENTION

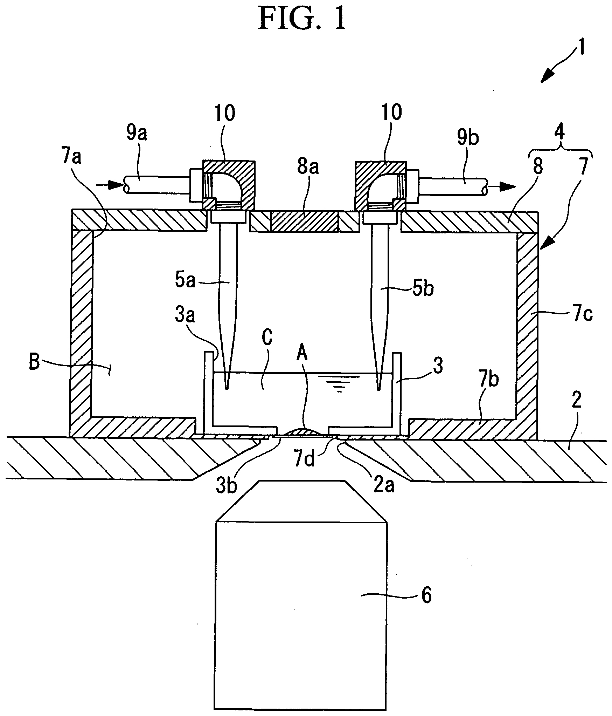

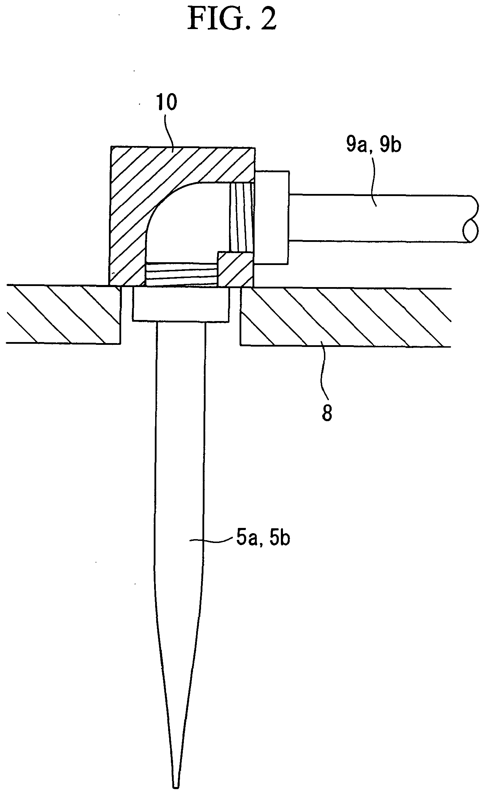

A culturing system 1 according to an embodiment of the present invention will now be described with reference to Figs. 1 and 2.

As shown in Fig. 1, the culturing system 1 according to this embodiment is mounted on a stage 2 of a microscope and includes a main container 4 accommodating a culture vessel 3 and nozzles 5a and 5b fixed to the main container 4.

The stage 2 of the microscope has a through-hole 2a at a position opposite an objective lens 6 disposed therebelow.

The culture vessel 3, which is a commercially available glass-bottomed dish, has an opening 3a facing upward and a glass slide 3b bonded to the underside thereof. A biological specimen A to be incubated, for example, a living cell, is placed on the glass slide 3b.

The main container 4 includes a base member 7 mounted on the stage 2 with an opening 7a thereof facing upward so as to seal off the through-hole 2a of the stage 2 and a lid member 8 disposed so as to seal off the opening 7a of the base member 7.

In addition, the main container 4 has a temperature-controlling unit, a CO₂-supplying unit, and a humidity-controlling unit (not shown) so that they can maintain an internal space B, formed by sealing off the top opening 7a of the base member 7 with the lid member 8, at a temperature of, for example, 37°C ± 0.5°C, a CO₂ concentration of, for example, 5% ± 1%, and a humidity of, for example, 95% ± 5%.

The base member 7 includes a bottom portion 7b mounted on the stage 2 and a peripheral wall 7c extending vertically upward from the edges of the bottom portion 7b.

A through-hole (transparent portion) 7d is provided in the bottom portion 7b of the base member 7 at a position where the through-hole 7d is sealed off by the underside of the culture vessel 3 after the culture vessel 3 is mounted.

The lid member 8 has a window portion 8a formed of a transparent material such as glass. The window portion 8a, which is used for transmission observation, transmits illumination light emitted from a light source (not shown) disposed vertically thereabove to illuminate the biological specimen A in the culture vessel 3 so that fluorescence emitted vertically downward from the biological specimen A or transmitted light can be observed through the objective lens 6.

In addition, the nozzles 5a and 5b are fixed to the lid member 8. The nozzles 5a and 5b, namely, the supply nozzle 5a and the suction nozzle 5b, are disposed under the lid member 8 so as to extend with the ends thereof facing vertically downward. The individual nozzles 5a and 5b, as shown in Fig. 2, are attachably/detachably attached by screw fitting to joints 10 that are fixed to the lid member 8 and that are connected to external tubes 9a and 9b.

When the lid member 8 is disposed at the position where it seals off the top opening 7a of the base member 7 with the two nozzles 5a and 5b attached to the joints 10, the nozzles 5a and 5b are inserted into the culture vessel 3 accommodated in the base member 7 through the top opening 3a of the culture vessel 3 and are positioned at predetermined distances from the bottom and side surfaces of the culture vessel 3.

The operation of the thus-configured culturing system (incubator) 1 according to this embodiment will be described below.

To examine the biological specimen A over a long period of time while incubating it in the culturing system 1 according to this embodiment, the culture vessel 3, which contains the biological specimen A together with a culture solution C, is accommodated in the base member 7 mounted on the stage 2. The top opening 7a of the base member 7 is then sealed off with the lid member 8, and the inner space B of the main container 4 is maintained at a predetermined incubation environment. The top opening 7a of the base member 7 does not necessarily have to be sealed off (in a completely hermetic manner) with the lid member 8 if the entire main container 4 is accommodated in another container maintained at the predetermined environment.

In this state, the ends of the supply nozzle 5a and the suction nozzle 5b are inserted into the culture vessel 3 through the opening 3a thereof and are positioned in the culture solution C. A pump (not shown) is then driven to supply fresh culture solution C through the supply nozzle 5a while draining the old culture solution C by suction through the suction nozzle 5b.

For epi-illumination observation, illumination light coming from below the stage 2 through the objective lens 6 passes through the through-hole 2a of the stage 2 and the through-hole 7d of the base member 7. The illumination light passes through the glass slide 3b at the bottom surface of the culture vessel 3 to irradiate the biological specimen A on the glass slide 3b. Fluorescence emitted from the biological specimen A passes through the glass slide 3b and the through-holes 2a and 7d and is observed after being collected by the objective lens 6.

For transmission illumination observation, on the other hand, illumination light coming from an upper light source enters the main container 4 through the window portion 8a to irradiate the biological specimen A through the culture solution C. Of the fluorescence produced in the biological specimen A, the fluorescence traveling downward through the glass slide 3b or transmitted light passes through the through-holes 2a and 7d and is observed after being collected by the objective lens 6. It is also possible to perform transmission observation using a detector for transmission detection disposed above the biological specimen A by illuminating the biological specimen A from below.

In this case, because the culturing system 1 according to this embodiment has the nozzles 5a and 5b, for supplying and draining the culture solution C, fixed to the main container 4 without contact with the culture vessel 3, even if the nozzles 5a and 5b vibrate due to, for example, pulsations of the pump during the supply or drainage of the culture solution C, the vibrations can be prevented from being transmitted to the culture vessel 3. This avoids problems such as vibration of the biological specimen A under magnification observation and blurring of the acquired image.

In this embodiment, additionally, because the nozzles 5a and 5b are fixed to the lid member 8, which is separated from the base member 7 in contact with the culture vessel 3, transmission of vibrations of the lid member 8 to the base member 7 is also inhibited. This advantageously avoids the problem of vibrations of the biological specimen A due to vibrations of the nozzles 5a and 5b in time-lapse examination, in which changes in the biological specimen A are examined over a long period of time, thus allowing accurate examination with a sharp image.

In this embodiment, additionally, because the nozzles 5a and 5b are attachably/detachably attached with the joints 10, the user can attach nozzles 5a and 5b of any shape depending on, for example, the type of biological specimen A under examination. For example, attaching tapered nozzles 5a and 5b minimizes the effect of the surface tension (the surface tension between the nozzles and the culture solution C) of the culture solution C supplied, thus allowing accurate adjustment of the liquid level.

In addition, long nozzles 5a and 5b may be used with the ends thereof disposed near the bottom surface of the culture vessel 3 so that the biological specimen A can be examined while being incubated with less culture solution C. This allows efficient utilization of an expensive reagent, for example, one for chemical stimulation.

Although the lid member 8 has the window portion 8a in this embodiment, the window portion 8a does not have to be provided if transmission illumination observation is not performed. In addition, although the nozzles 5a and 5b are attachably/detachably attached, they do not have to be attachable/detachable.

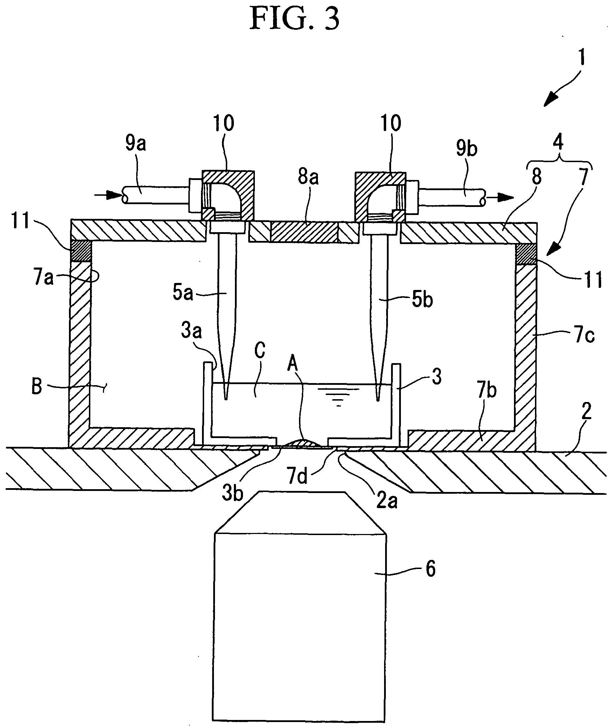

As shown in Fig. 3, additionally, a damper 11 is preferably disposed between the base member 7 and the lid member 8. The damper 11 is formed of a viscoelastic material such as polyisoprene rubber. The intervening damper 11 absorbs vibrations transmitted from the nozzles 5a and 5b to the lid member 8 to inhibit transmission of vibrations to the base member 7, thus further alleviating vibration of the culture vessel 3.

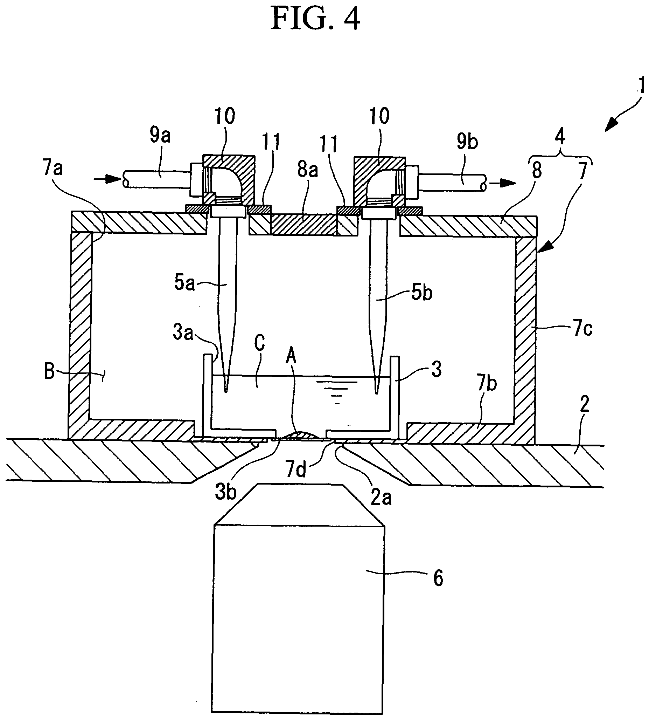

As shown in Fig. 4, the damper 11 may also be disposed between the joints 10 and the lid member 8. This inhibits transmission of vibrations from the nozzles 5a and 5b to the lid member 8, thus alleviating vibration of the culture vessel 3.

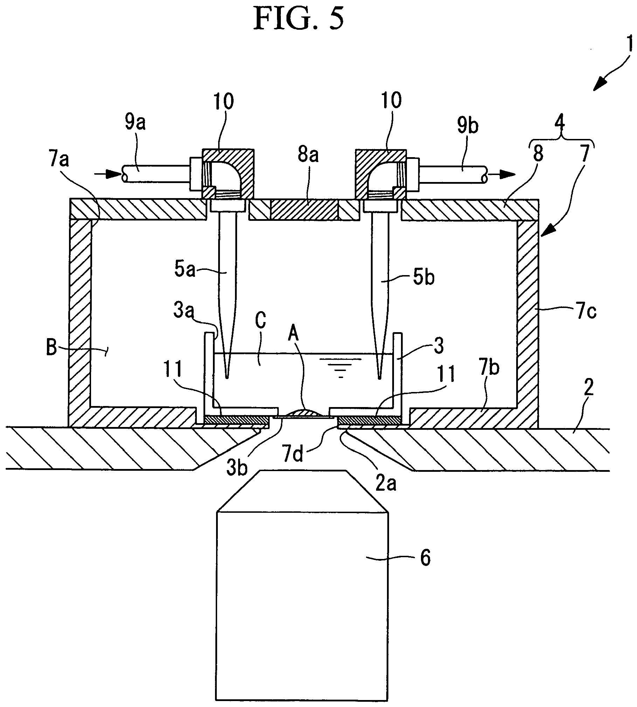

As shown in Fig. 5, the damper 11 may also be disposed between the bottom portion 7b of the base member 7 and the culture vessel 3. This inhibits transmission of vibrations from the base member 7 to the culture vessel 3 even if vibrations of the nozzles 5a and 5b are transmitted to the lid member 8 and the base member 7, thus alleviating vibration of the culture vessel 3. Naturally, the dampers 11 disposed at the above positions may be used in combination.

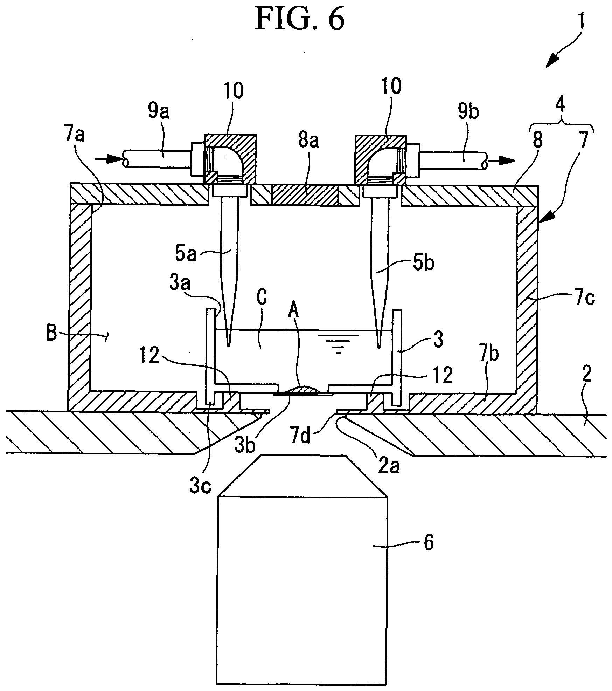

As shown in Fig. 6, additionally, a support 12 for the culture vessel 3 may be provided on the bottom portion 7b of the base member 7. The support 12 may be disposed slightly inside the periphery of the culture vessel 3 so as to protrude vertically upward from the bottom portion 7b in a rectangular cross section.

Some types of culture vessel 3, as shown in Fig. 6, have a leg portion 3c protruding from the outermost periphery thereof, and the height of the leg portion 3c varies depending on the type of culture vessel 3. Instead of bringing the leg portion 3c into contact with the bottom portion 7b of the base member 7, the support 12 can be brought into contact with the underside of the culture vessel 3 to maintain the level of the underside of the culture vessel 3, that is, the level of the biological specimen A, at substantially the same level irrespective of the type of culture vessel 3.

This allows the level of the ends of the nozzles 5a and 5b to be always kept constant relative to that of the biological specimen A for different types of culture vessel 3, thus providing identical observation conditions.

## Claims

1. A culturing system (1) comprising:
a main container (4) which has an inner space (B) maintained at a predetermined incubation environment, and which accommodates a culture vessel (3) therein, wherein the culture vessel (3) has an opening (3a) facing upward, and is adapted to contain a biological specimen (A); and
a nozzle (5a, 5b) fixed to the main container (4) and inserted into the culture vessel (3) through the opening (3a) of the culture vessel (3) to supply or drain a liquid into or from the culture vessel (3);
wherein the main container (4) includes a transparent portion (7d) through which the biological specimen (A) in the main container (4) can be externally observed,
**characterized in that** the nozzle (5a, 5b) is only fixed to the main container (4) without being in direct contact with the culture vessel (3).

2. The culturing system according to Claim 1, wherein
the main container (4) includes a base member (7) having a base member opening (7a) facing upward and a lid member (8) sealing off the base member opening (7a);
the base member (7) including a bottom portion (7b) on which the culture vessel (3) is mounted and a peripheral wall (7c) extending upward from the bottom portion (7b);
wherein the nozzle (5a, 5b) is fixed to the lid member (8).

3. The culturing system according to Claim 2, wherein a damper (11) is disposed between the base member (7) and the lid member (8) to inhibit transmission of vibrations therebetween.

4. The culturing system according to Claim 1 or 2, wherein a damper (11) is disposed between the main container (4) and the nozzle (5a, 5b) to inhibit transmission of vibrations therebetween.

5. The culturing system according to Claim 2, wherein
a support (12) is provided on the bottom portion of the base member (7) so as to support the culture vessel (3); and
a damper (11) is disposed between the support (12) and the culture vessel (3) to inhibit transmission of vibrations therebetween.

6. The culturing system according to Claim 2, wherein a support (12) is provided on the bottom portion (7b) of the base member (7) so as to support the culture vessel (3) in contact with the underside of the culture vessel (3).

7. The culturing system according to one of Claims 1 to 6, wherein the nozzle (5a, 5b) is attachably/detachably attached to the main container.

8. The culturing system according to any of Claims 1 to 7, wherein the nozzle comprises a supply nozzle (5a) and a suction nozzle (5b).

## Patentansprüche

1. Kultivierungssystem (1), das umfasst:
einen Hauptbehälter (4) mit einem inneren Raum (B), der bei einer vorbestimmten Inkubationsumgebung gehalten wird und der ein Kulturgefäß (3) darin aufnimmt, wobei das Kulturgefäß (3) eine nach oben weisende Öffnung (3a) hat und dazu eingerichtet ist, eine biologische Probe (A) zu enthalten; und
eine Düse (5a, 5b), die an dem Hauptbehälter (4) befestigt und durch die Öffnung (3a) des Kulturgefäßes (3) in das Kulturgefäß (3) eingeführt ist, um eine Flüssigkeit in das Kulturgefäß (3) zuzuführen oder daraus abzuführen;
wobei der Hauptbehälter (4) einen transparenten Abschnitt (7d) umfasst, durch den die biologische Probe (A) in dem Hauptbehälter (4) von außen beobachtet werden kann,
**dadurch gekennzeichnet, dass** die Düse (5a, 5b) nur an dem Hauptbehälter (4) befestigt ist, ohne in direktem Kontakt mit dem Kulturgefäß (3) zu sein.

2. Kultivierungssystem nach Anspruch 1, wobei
der Hauptbehälter (4) ein Basiselement (7) mit einer nach oben weisenden Basiselementöffnung (7a) und ein die Basiselementöffnung (7a) abdichtendes Deckelelement (8) umfasst;
das Basiselement (7) einen Bodenabschnitt (7b), an dem das Kulturgefäß (3) angebracht ist, und eine sich von dem Bodenabschnitt (7b) nach oben erstreckende Umfangswand (7c) umfasst;
wobei die Düse (5a, 5b) an dem Deckelelement (8) befestigt ist.

3. Kultivierungssystem nach Anspruch 2, wobei ein Dämpfer (11) zwischen dem Basiselement (7) und dem Deckelelement (8) angeordnet ist, um die Übertragung von Vibrationen zwischen diesen zu verhindern.

4. Kultivierungssystem nach Anspruch 1 oder 2, wobei ein Dämpfer (11) zwischen dem Hauptbehälter (4) und der Düse (5a, 5b) angeordnet ist, um die Übertragung von Vibrationen zwischen diesen zu verhindern.

5. Kultivierungssystem nach Anspruch 2, wobei
ein Träger (12) an dem Bodenabschnitt des Basiselements (7) vorgesehen ist, um das Kulturgefäß (3) abzustützen; und
ein Dämpfer (11) zwischen dem Träger (12) und dem Kulturgefäß (3) angeordnet ist, um die Übertragung von Vibrationen zwischen diesen zu verhindern.

6. Kultivierungssystem nach Anspruch 2, wobei ein Träger (12) an dem Bodenabschnitt (7b) des Basiselements (7) vorgesehen ist, um das Kulturgefäß (3) in Kontakt mit der Unterseite des Kulturgefäßes (3) abzustützen.

7. Kultivierungssystem nach einem der Ansprüche 1 bis 6, wobei die Düse (5a, 5b) befestigbar/lösbar an dem Hauptbehälter befestigt ist.

8. Kultivierungssystem nach einem der Ansprüche 1 bis 7, wobei die Düse eine Zufuhrdüse (5a) und eine Saugdüse (5b) umfasst.

## Revendications

1. Système de culture (1) comprenant :
un contenant principal (4) qui comporte un espace interne (B) maintenu dans un environnement d'incubation prédéterminé, et qui reçoit un récipient de culture (3) dans celui-ci, dans lequel le récipient de culture (3) comporte une ouverture (3a) en regard vers le haut, et est adapté pour contenir un spécimen biologique (A) ; et
une buse (5a, 5b) fixée au contenant principal (4) et insérée dans le récipient de culture (3) à travers l'ouverture (3a) du récipient de culture (3) pour délivrer ou drainer un liquide dans ou depuis le récipient de culture (3) ;
dans lequel le contenant principal (4) comprend une partie transparente (7d) à travers laquelle le spécimen biologique (A) dans le contenant principal (4) peut être observé depuis l'extérieur,
**caractérisé en ce que** la buse (5a, 5b) est uniquement fixée au contenant principal (4) sans être en contact direct avec le récipient de culture (3).

2. Système de culture selon la revendication 1, dans lequel
le contenant principal (4) comprend un élément de base (7) comportant une ouverture (7a) d'élément de base en regard vers le haut et un élément de couvercle (8) fermant hermétiquement l'ouverture (7a) d'élément de base ;
l'élément de base (7) comprenant une partie de fond (7b) sur laquelle le récipient de culture (3) est monté et une paroi périphérique (7c) s'étendant vers le haut depuis la partie de fond (7b) ;
dans lequel la buse (5a, 5b) est fixée à l'élément de couvercle (8).

3. Système de culture selon la revendication 2, dans lequel un amortisseur (11) est disposé entre l'élément de base (7) et l'élément de couvercle (8) pour empêcher la transmission des vibrations entre eux.

4. Système de culture selon la revendication 1 ou 2, dans lequel un amortisseur (11) est disposé entre le contenant principal (4) et la buse (5a, 5b) pour empêcher la transmission des vibrations entre eux.

5. Système de culture selon la revendication 2, dans lequel
un support (12) est prévu sur la partie de fond de l'élément de base (7) de façon à supporter le récipient de culture (3) ; et
un amortisseur (11) est disposé entre le support (12) et le récipient de culture (3) pour empêcher la transmission des vibrations entre eux.

6. Système de culture selon la revendication 2, dans lequel le support (12) est prévu sur la partie de fond (7b) de l'élément de base (7) de façon à supporter le récipient de culture (3) en contact avec la face inférieure du récipient de culture (3).

7. Système de culture selon l'une quelconque des revendications 1 à 6, dans lequel la buse (5a, 5b) est attachée de manière attachable/détachable au contenant principal.

8. Système de culture selon l'une quelconque des revendications 1 à 7, dans lequel la buse comprend une buse d'alimentation (5a) et une buse d'aspiration (5b).
